# EUROPÄISCHE PATENTSCHRIFT

(11) **EP 0 815 814 B1**
(45) Veröffentlichungstag und Bekanntmachung des Hinweises auf die Patenterteilung: **18.12.2002**
(21) Anmeldenummer: 97104933.3
(22) Anmeldetag: 22.03.1997
(51) Int. Cl.: A61F 9/02, C09K 3/18

(54) **Arbeitsschutzbrille**
Working safety glass
Lunettes protectrices du travail

(30) Priorität: 04.07.1996 DE 29611652 U
(43) Veröffentlichungstag der Anmeldung: 07.01.1998
(73) Patentinhaber: Uvex Arbeitsschutz GmbH, 90766 Fürth (DE)
(72) Erfinder: Wiedner, Klaus, 90768 Fürth/Bay (DE)
(74) Vertreter: Schneck, Herbert, Dipl.-Phys., Dr.

(56) Entgegenhaltungen:
- EP-A- 0 529 202
- WO-A-94/03831
- DATABASE WPI Section Ch, Week 9421 Derwent Publications Ltd., London, GB; Class A89, AN 94-173885 XP002057241 & JP 06 116 429 A (ITOCHU FINE CHEM KK)

## Beschreibung

Die Erfindung richtet sich auf eine Arbeitsschutzbrille gemäß dem Oberbegriff von Anspruch 1.

Bekannte derartige Arbeitsschutzbrillen weisen einen sich auf die Stirn des Benutzers zu erstreckenden, etwa der Stirnkontur folgenden oberen horizontalen Abschnitt auf. Allerdings kann die Innenkante eines solchen Abschnitts der individuellen Stirnform des Benutzers immer nur annähernd folgen, so daß unterschiedlich große Freiräume zwischen Brille und Stirn verbleiben, durch die Spritzer und Partikel, insbesondere Staub, eindringen können.

Man hat deshalb schon versucht, eine weitergehende Individualisierung dadurch zu erreichen, daß man sich über einen Teil der Oberseite erstrekkende Aufsteckteile aus hartem Kunststoff vorgesehen hat. Hierdurch wird zwar eine gewisse Verbesserung erreicht, eine wirklich bündige Abdekkung der Oberseite im Hinblick auf eine Staubdichtigkeit wird aber nicht erzielt.

Aus WO 94/03831 ist eine gattungsgemäße Schutzbrille bekannt, bei welcher die Abdeckung aus Schaumstoff besteht, wobei die Abdeckung durch Kleben festgelegt wird oder einen Schlitz aufweist, um sie aufzustecken und beispielsweise durch Rippen mechanisch festzuhalten. Vergleichbare Schaumstoffstreifen sind auch aus US 5,495,303 bekannt.

Hiervon ausgehend liegt der Erfindung die Aufgabe zugrunde, eine Schutzbrille der eingangs genannten Art so auszugestalten, daß bei hohem Tragekomfort die Augen des Benutzers zuverlässig gegen das Eindringen von Staub, Tropfen oder dergleichen geschützt werden, wobei eine einfache Herstellung und eine zuverlässige Festlegung der Abdeckung gewährleistet sein sollen.

Diese Aufgabe wird erfindungsgemäß gelöst gemäß dem kennzeichnendenden Teil von Anspruch 1. Hierdurch wird eine gleichermaßen feste wie einfach zu realisierende Befestigung und Ausgestaltung erreicht.

Die Scheibe besteht vorzugsweise aus TPE oder TPU. Hierbei handelt es sich um Kunststoffe, welche sich gut mit dem Scheibenmaterial bzw. dem Coating des Scheibenmaterials verbinden.

Die eigentliche Abdeckung der Oberseite wird relativ dünn ausgebildet und weist beispielsweise eine Dicke von etwa 1 mm auf. Sie besteht aus massivem Kunststoff, also nicht aus Schaumstoffmaterial, und stellt hierdurch sicher, daß von der Oberseite her nichts an die Augen des Benutzers gelangt. Andererseits wird aufgrund der relativ geringen Dicke eine solche Weichheit erreicht, daß ein gutes Anschmiegen an die Stirnkonfiguration des Benutzers erreichbar ist. Weiterhin ist es aufgrund der glatten Oberfläche des Kunststoffes möglich, die Brille gut zu reinigen. Die herkömmlicherweise verwendeten Schaumstoffaufsätze sind insoweit erheblich nachteiliger. Andererseits kann die erfindungsgemäße Abdeckung wiederum so dünn ausgestaltet werden, daß trotz der Verwendung eines massiven Kunststoffs die erforderliche Weichheit erzielt wird.

In weiterer Ausgestaltung der Erfindung kann vorgesehen sein, daß an dem inneren Rand des sich nach hinten erstreckenden Scheibenansatzes ein sich nach oben wegerstreckender, vorzugsweise bogenförmiger Arretierabschnitt angeformt ist, der eine gute Verbindung zwischen dem Scheibenmaterial und dem angespritzten Kunststoffinaterial ermöglicht.

Günstigerweise werden Scheibenabdeckungen im Zwei-Komponenten-Spritzverfahren hergestellt.

Bei einer bevorzugten Ausführungsform ist vorgesehen, daß auf der Scheibe im Nasenbereich ein Nasenpolster und/oder ein Kratzschutz für die Scheibe angespritzt sind, welche einstückig mit der erfindungsgemäßen Abdeckung ausgebildet und in einem Spritzvorgang mit dieser hergestellt sein können.

Aufgrund der erfindungsgemäßen Abdeckung, die der Stirn des Benutzers besonders dicht anliegt, ist es besonders wichtig, daß die Scheibe eine hochwirksame Antibeschlag-Beschichtung aufweist. Insoweit ist vorgesehen, daß die Antibeschlag-Beschichtung an der Scheibeninnenseite in Kombination eine hydrophile Polyurethankomponente, welche wasserabsorbierend wirkt, und ein oberflächenaktives Tensid umfaßt, welches die Tropfenbildung verhindert und die Ausbildung einer gleichförmigen Wasserschicht bewirkt. Die Beschichtung wird also im Ein-Topf-Verfahren hergestellt und dementsprechend werden sowohl die Polyurethankomponente als auch das Tensid in einem Arbeitsgang als einzige Beschichtung aufgebracht.

Nachfolgend wird die Erfindung anhand eines bevorzugten Ausführungsbeispiels in Verbindung mit der Zeichnung näher erläutert. Dabei zeigen:
- Fig. 1: eine perspektivische Ansicht einer Scheibe einer erfindungsgemäßen Arbeitsschutzbrille mit zugeordneter stirnseitiger Abdeckung von vorne gesehen nach Art einer Explosionsdarstellung,
- Fig. 2: die Brille bzw. Scheibe nach Fig. 1 im Gebrauchszustand und
- Fig. 3: eine Fig. 2 entsprechende Darstellung einer abgewandelten Ausführungsform.

Eine in der Zeichnung dargestellte Arbeitsschutzbrille umfaßt eine einstükkige Kunststoffscheibe 1 mit einem vorderen Sichtbereich 2 und einem Nasenauflagebereich 3, wobei sich von dem Sichtbereich 2 seitlich nach hinten Ansätze 4 mit angespritzten Lageransätzen 5 für in der Zeichnung nicht dargestellte Brillenbügel erstrecken. Längs der Oberkante 6 des Sichtbereiches 2 erstreckt sich ein im Gebrauchszustand horizontaler Ansatz 7, dessen Innenkante 8 etwa der Krümmung der Stirn des Benutzers folgt. Von der Oberkante 6 vertikal nach oben erstreckt sich ein bogenförmig geformter Arretieransatz 9 längs des Mittelbereiches der Kante 6.

Ein oberer Aufsatz 10 ist einstückig aus relativ weichem Kunststoff gespritzt und umfaßt ein horizontales Flächenelement 11 und ein längs dessen Vorderkante verlaufendes Rahmenelement 12, welches die seitlichen Ansätze 4 mit Abschnitten 13 übergreift.

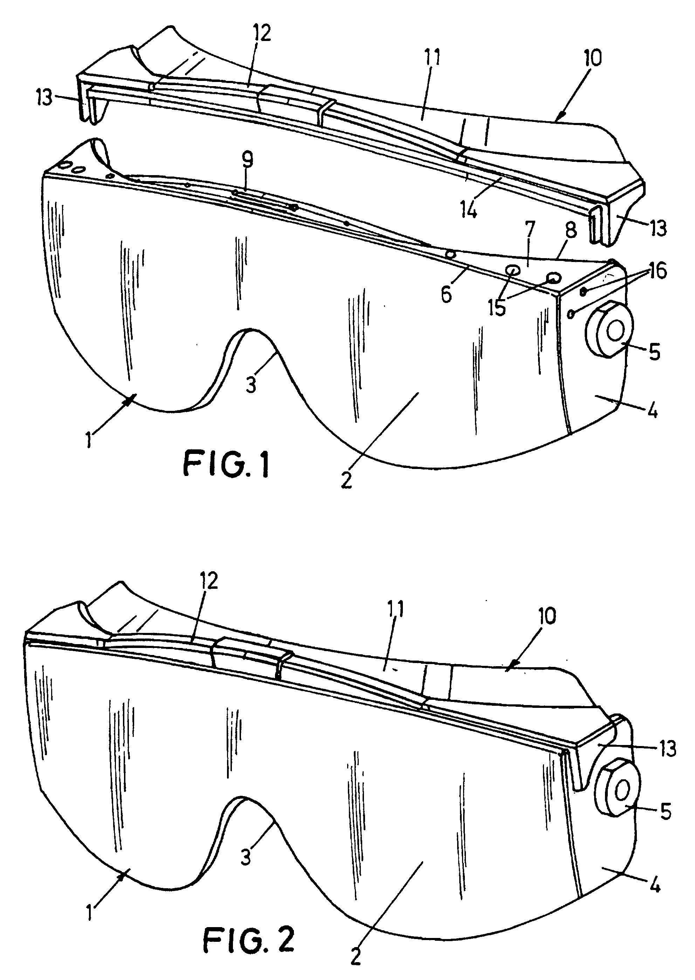

Der Aufsatz 10 weist also eine Nut 14 auf. Allerdings wird der Aufsatz 10 in der in Fig. 1 dargestellten isolierten Form bei der spritztechnischen Herstellung so nicht realisiert, weil die Scheibe in der Form liegend mit dem den Aufsatz 10 ausbildenden relativ weichen Kunststoff umspritzt wird. Um eine feste Verbindung zwischen diesem weichen Kunststoff und dem Scheibenmaterial zu erzielen, sind in den Ansätzen 7 bzw. 4 Durchbrechungen 15 bzw. 16 vorgesehen, so daß der Kunststoff des Aufsatzes 10 diese durchdringt und der Aufsatz damit auch formschlüssig festgelegt ist. Es entsteht dementsprechend ein Fertigprodukt, wie es in Fig. 2 dargestellt ist.

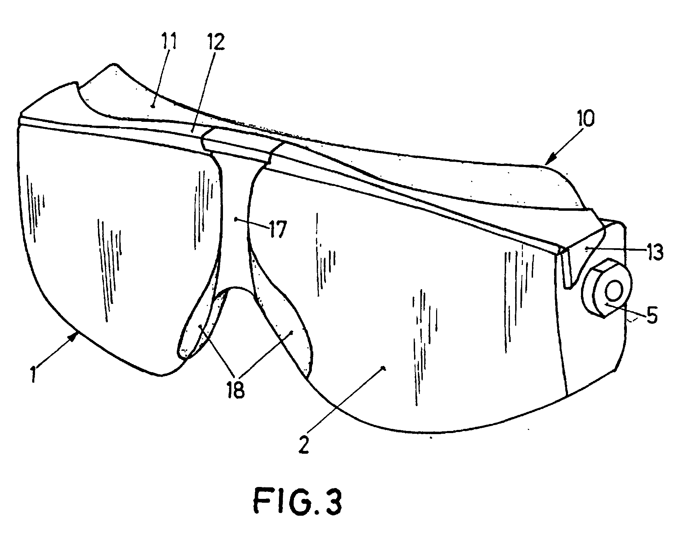

Die Ausführungsform nach Fig. 3 unterscheidet sich von derjenigen nach Fig. 1 und 2 dadurch, daß an den Rahmen 12 des Aufsatzes 10 noch ein Kratzschutz 17 angespritzt ist, der praktisch parallel zum Nasenbereich an der Außenseite der Scheibe 1 verläuft und in Nasenauflageteile 18 mündet.

## Patentansprüche

1. Arbeitsschutzbrille mit einer einstückigen Kunststoffscheibe und einer an dieser festlegbaren stirnseitigen Abdeckung aus relativ weichem Kunststoff, welche eine zur Scheibe hin offene Nut aufweist, **dadurch gekennzeichnet, daß** die Abdeckung (10) an die Scheibe (1) angespritzt ist, daß das Rahmenteil um Ansätze (4 bzw. 7) der Scheibe (1) gespritzt ist, und daß die Ansätze (4 bzw. 7) Durchbrechungen (15 bzw. 16) aufweisen, um eine feste Verbindung zwischen dem weichen Kunststoff der Abdeckung (10) und dem Scheibenmaterial zu erreichen.

2. Arbeitsschutzbrille nach Anspruch 1, **dadurch gekennzeichnet, daß** die stirnseitige Abdeckung (10) aus TPE oder TPU hergestellt ist.

3. Arbeitsschutzbrille nach Anspruch 1, **dadurch gekennzeichnet, daß** an dem inneren Rand (8) des sich nach hinten erstreckenden Scheibenansatzes (7) ein sich nach oben weg erstreckender, vorzugsweise bogenförmiger Arretierabschnitt (9) angeformt ist.

4. Arbeitsschutzbrille nach Anspruch 1, **dadurch gekennzeichnet, daß** auf der Scheibe (1) im Nasenbereich ein Nasenpolster (18) und/oder Kratzschutz (17) für die Scheibe (1) angespritzt ist.

5. Arbeitsschutzbrille nach Anspruch 4, **dadurch gekennzeichnet, daß** das Nasenpolster (17) und/oder Kratzschutz (18) mit der Abdeckung (10) einstückig gespritzt ist.

6. Arbeitsschutzbrille nach Anspruch 1, **gekennzeichnet durch** eine Antibeschlag-Beschichtung an der Scheibeninnenseite umfassend in Kombination eine hydrophile Polyurethankomponente, welche wasserabsorbierend wirkt, und ein oberflächenaktives Tensid, welches die Tropfenbildung verhindert und die Ausbildung einer gleichförmigen Wasserschicht bewirkt.

## Claims

1. Industrial safety glasses comprising a one-piece plastics sight piece and a top of comparatively soft plastics on the forehead side which can be fixed to the sight piece and has a groove open toward the sight piece **characterized in that** the top (10) is injection-molded on the sight piece (1), that the frame element (12) is injection-molded around joined-on pieces (4 and 7, respectively) of the sight piece (1) and that the joined-on pieces (4) have openings (15 and 16, respectively), in order to reach a firm compound between the soft plastic of the top (10) and the sight piece material.

2. Industrial safety glasses according to claim 1, **characterized in that** the top (10) on the forehead side is made of TPE or TPU.

3. Industrial safety glasses according to claim 1, **characterized in that** a preferably arc-shaped arresting section (9) extending upwards is injection-molded on the inner edge (8) of the piece (7) joined on the sight piece and extending backwards.

4. Industrial safety glasses according to claim 1, **characterized in that** a nose saddle (18) and/or protector against scratching (18) of the sight piece (1) is applied to the nose portion of the sight piece (1) by injection-molding.

5. Industrial safety glasses according to claim 4, **characterized in that** the nose saddle (17) and/or protector against scratching (18) is injection-molded, forming one piece with the top (10).

6. Industrial safety glasses according to claim 1, **characterized by** an antifogging layer on the inside of the sight piece comprising a combination of a hydrophilic polyurethane component, which has a water-absorbing effect, and a surface-active tenside, which prevents drop formation and causes the formation of a water film.

## Revendications

1. Lunettes protectrices de travail comprenant une visière en matière plastique d'une seule pièce et une couverture frontale en matière plastique relativement souple qui peut se fixer sur celle-ci, présentant une rainure ouverte en direction de la visière, **caractérisées en ce que** la couverture (10) est moulée par injection sur la visière (1), **en ce que** la partie formant cadre est injectée autour des pièces ajoutées (4 ou 7) de la visière (1) et **en ce que** les pièces ajoutées (4 ou 7) présentent des trous (15 ou 16) pour assurer une liaison solide entre la matière plastique souple de la couverture (10) et le matériau de la visière.

2. Lunettes protectrices de travail selon la revendication 1, **caractérisées en ce que** la couverture frontale (10) est fabriquée en TPE ou en TPU.

3. Lunettes protectrices de travail selon la revendication 1, **caractérisées en ce qu'**un segment d'arrêt (9) s'étendant vers le haut, de préférence de forme arquée, est moulé sur le bord interne (8) de la pièce ajoutée de la visière (7) s'étendant vers l'arrière.

4. Lunettes protectrices de travail selon la revendication 1, **caractérisées en ce qu'**un rembourrage pour le nez (18) est moulé par injection sur la visière (1) dans la zone du nez et/ou **en ce que** une protection anti-rayures (17) pour la visière (1) est moulée par injection.

5. Lunettes protectrices de travail selon la revendication 4, **caractérisées en ce que** le rembourrage pour le nez (17) et/ou la protection anti-rayures (18) sont injectés d'une seule pièce avec la couverture (10).

6. Lunettes protectrices de travail selon la revendication 1, **caractérisées par** un revêtement antibuée comprenant sur la face interne de la visière, en combinaison, un composant polyuréthane hydrophile ayant pour effet d'absorber l'eau et un agent tensioactif qui empêche la formation de gouttes et assure le développement d'une couche d'eau uniforme.
